# FASCICULE DE BREVET EUROPEEN

(11) **EP 2 310 141 B1**
(45) Date de publication et mention de la délivrance du brevet: **28.01.2015**
(21) Numéro de dépôt: 09766075.7
(22) Date de dépôt: 16.06.2009
(51) Int. Cl.: B05B 11/00, A61M 15/00, A61M 15/08, B65D 83/38, B65D 83/20

(54) **DISPOSITIF DE DISTRIBUTION DE PRODUIT FLUIDE**
AUFTRAGVORRICHTUNG EINES FLUIDS
FLUID PRODUCT DISPENSING DEVICE

(30) Priorité: 17.06.2008 FR 0853995
(43) Date de publication de la demande: 20.04.2011
(73) Titulaire: Aptar France SAS, 27110 Le Neubourg (FR)
(72) Inventeur: LEMANER, François, F-27400 La Vallee Montaure (FR)
(74) Mandataire: CAPRI
(86) Numéro de dépôt international: PCT/FR2009/051137
(87) Numéro de publication internationale: WO 2009/153513

(56) Documents cités:
- EP-A- 1 974 827
- WO-A-01/03851
- WO-A-2005/075105
- WO-A-2006/005962
- WO-A1-2009/068877
- FR-A1- 2 812 826
- US-A- 5 487 489
- US-A1- 2007 131 717

## Description

La présente invention concerne un dispositif de distribution de produit fluide, et plus particulièrement un dispositif de pulvérisation nasale d'un produit pharmaceutique.

Les dispositifs de distribution de produit fluide sont bien connus dans l'état de la technique. Ils comportent généralement un réservoir contenant le produit fluide sur lequel est assemblé un organe de distribution, par exemple une pompe ou une valve, qui est généralement actionné au moyen d'une tête de distribution pour distribuer de manière sélective le produit contenu à l'intérieur dudit réservoir. La tête de distribution comporte un orifice de distribution à travers lequel le produit va être pulvérisé, par exemple dans le nez de l'utilisateur dans le cas d'un dispositif de pulvérisation nasale. De nombreux dispositifs de ce type sont actionnés manuellement par l'utilisateur en déplaçant axialement l'un contre l'autre le réservoir et la tête de distribution, ce qui a pour effet d'actionner l'organe de distribution. Ce type de dispositif présente toutefois des inconvénients, notamment lorsque le dispositif est du type à pulvérisation nasale, car la force axiale exercée par l'utilisateur pour actionner le dispositif induit un risque de déplacement de la tête de distribution à l'intérieur de la narine de l'utilisateur, avec des risques de blessure et/ou de distribution incomplète ou inadéquate du produit au moment de l'actionnement. Pour remédier à ce problème, des dispositifs d'actionnement latéral ont été proposés, comportant généralement un levier monté pivotant sur un corps et dont une partie interne est adaptée à coopérer avec l'un parmi la tête de distribution ou le réservoir pour déplacer cet élément contre l'autre et ainsi actionner l'organe de distribution. Ces dispositifs induisent toutefois des contraintes radiales lors de l'actionnement qui peuvent aussi avoir des influences négatives sur la pulvérisation du produit dans la narine de l'utilisateur. Le document WO 2005/075105 décrit un dispositif de l'art antérieur. Le document WO 2009/068877 A1 décrit un dispositif selon le préamble de la revendication 1.

La présente invention a pour but de fournir un dispositif de distribution de produit fluide qui ne reproduit pas les inconvénients susmentionnés.

La présente invention a aussi pour but de fournir un dispositif de distribution de produit fluide, notamment de pulvérisation nasale, qui garantit un actionnement sûr et fiable du dispositif à chaque actionnement, sans risque de blessure pour l'utilisateur.

Plus particulièrement, la présente invention a pour but de fournir un dispositif de distribution de produit fluide qui soit simple et peu coûteux à fabriquer et à assembler.

La présente invention a donc pour objet un dispositif de distribution de produit fluide selon la revendication 1.

Avantageusement, ledit au moins un élément d'actionnement comporte une zone de contact arrondie coopérant avec ladite surface de guidage sur toute la course d'actionnement.

Avantageusement, ladite surface de guidage est solidaire de la tête de distribution incorporant l'orifice de distribution.

Avantageusement, ledit au moins un élément d'actionnement comporte un oeillet monté autour de ladite projection, ledit élément d'actionnement pivotant autour de ladite projection pendant l'actionnement.

Avantageusement, ledit élément d'appui comporte une zone d'appui sur laquelle l'utilisateur appuie lors de l'actionnement.

Avantageusement, ledit axe (A) est plus éloigné de l'orifice de distribution que ledit autre axe (B).

Avantageusement, ledit axe (A) est plus proche de l'orifice de distribution que ledit autre axe (B).

Avantageusement, ledit élément d'actionnement comporte une zone d'appui sur laquelle l'utilisateur appuie lors de l'actionnement.

Avantageusement, ledit corps comporte un manchon de guidage axial pour guider le réservoir lors de l'actionnement.

Ces caractéristiques et avantages et d'autres de la présente invention apparaîtront plus clairement au cours de la description détaillée suivante de plusieurs modes de réalisation de celle-ci, faite en référence aux dessins joints, donnés à titre d'exemples non limitatifs, sur lesquels :
- les figures 1 et 2 représentent des vues schématiques d'un premier mode de réalisation de la présente invention, respectivement en position de repos et en position d'actionnement ;
- les figures 3 et 4 représentent des vues schématiques d'un mode de réalisation qui ne fait pas partie de la présente invention, respectivement en position de repos et en position d'actionnement ;
- Les figures 5 et 6 représentent des vues schématiques d'un mode de réalisation qui ne fait pas partie de la présente invention respectivement en position de repos et en position d'actionnement ; et
- les figures 7 et 8 représentent des vues schématiques d'un second mode de réalisation de la présente invention, respectivement en position de repos et en position d'actionnement.

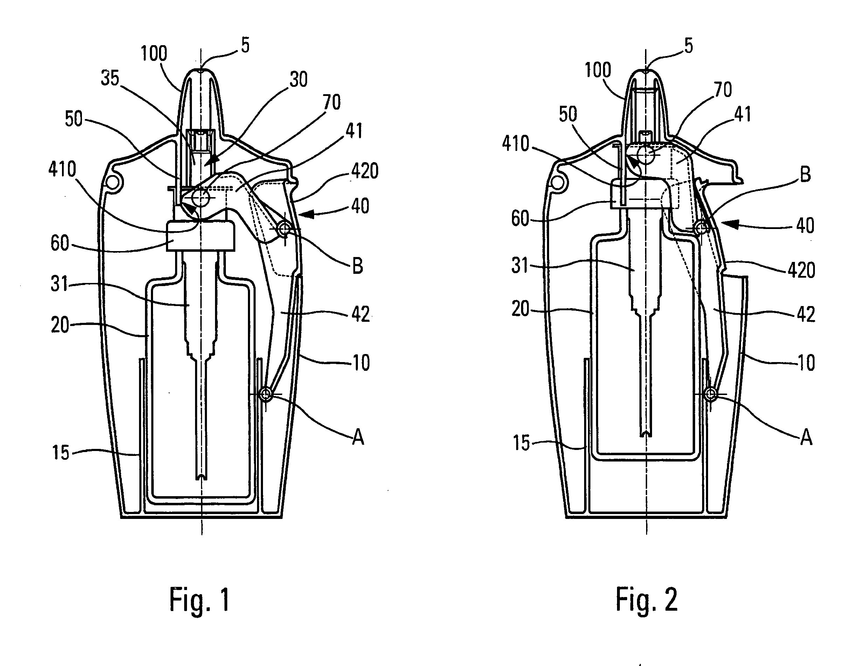

En se référant aux figures 1 et 2, qui illustrent un premier mode de réalisation avantageux de l'invention, le dispositif de distribution de produit fluide comporte un corps 10 dans lequel est assemblé un réservoir 20 qui contient du produit fluide, notamment du produit pharmaceutique destiné à être pulvérisé dans le nez d'un utilisateur. Un organe de distribution 30, tel qu'une pompe ou une valve, est assemblé sur ledit réservoir 20, au moyen d'une bague de fixation 60. Cet organe de distribution comporte un corps d'organe de distribution 31, tel qu'un corps de pompe ou un corps de valve, et un organe mobile 35, tel qu'une tige de piston ou une soupape de valve, qui est monté coulissant dans ledit corps d'organe de distribution. De manière classique, l'organe mobile 35 est enfoncé à l'intérieur du corps d'organe de distribution 31, pour actionner ledit organe de distribution 30. Ceci est représenté très schématiquement sur les dessins, avec l'organe mobile 35 en position haute sur les figures 1, 3, 5 et 7 (position de repos) et en position enfoncée sur les figures 2, 4, 6 et 8 (position d'actionnement). Avantageusement, une tête de distribution 100 est assemblée sur ledit organe de distribution 30, ladite tête de distribution 100 incorporant un orifice de distribution 5 à travers lequel le produit fluide sera distribué. Dans les exemples représentés, la tête 100 fait partie ou est solidaire du corps 10 du dispositif. Elle peut être réalisée d'une pièce monobloc avec ledit corps.

Le dispositif comporte un système d'actionnement latéral 40 solidaire du corps 10 et adapté à coopérer avec un élément solidaire dudit réservoir 20 tel que la bague de fixation 60 qui fixe l'organe de distribution 30 sur le réservoir 20. Ce système d'actionnement latéral 40 comporte un élément d'actionnement 41. Dans l'exemple des figures 1 et 2, cet élément d'actionnement 41 est monté pivotant sur un élément d'appui 42, autour d'un axe B. Cet élément d'appui 42 est lui monté pivotant sur le corps 10, autour d'un axe A. Le mode de réalisation des figures 1 et 2 comporte donc deux éléments pivotants interconnectés, l'utilisateur appuyant sur l'élément d'appui 42, de préférence via une zone d'appui 420 pour faire pivoter cet élément d'appui 42 par rapport au corps 10 autour de l'axe A, ce pivotement de l'élément d'appui 42 provoquant le pivotement de l'élément d'actionnement 41 par rapport audit élément d'appui 42 autour de l'axe B, et ainsi l'actionnement de l'organe de distribution 30. L'élément d'actionnement 41 coopère avec une projection 70, qui est solidaire d'un élément solidaire de reservoir. Selon l'invention cette projection 70 est formée sur une bague 60 qui est assemblée sur ledit réservoir, cette bague 60 servant à fixer l'organe de distribution 30 sur le réservoir.

Selon l'invention, le corps 10 du dispositif comporte au moins une surface de guidage 50 qui sert à guider l'élément d'actionnement 41 pendant sa course d'actionnement, c'est-à-dire pendant son pivotement lorsque l'utilisateur actionne le système d'actionnement latéral 40. Cette surface de guidage 50, comme visible sur les figures 1 et 2 notamment, est verticale et axiale, c'est-à-dire qu'elle est sensiblement parallèle à l'axe de déplacement de l'organe mobile 35 dans le corps d'organe de distribution 31 lors de l'actionnement de l'organe de distribution 31 lors de l'actionnement dudit organe de distribution 30. La surface de guidage 50 est de préférence plane. Elle est fixe par rapport à la tête de distribution pendant l'actionnement. Cette surface de guidage sert ainsi à diminuer, et notamment à supprimer les contraintes radiales exercées par le système d'actionnement latéral 40 lors de l'actionnement du dispositif. De cette manière, il n'y a plus de risque que l'utilisateur au moment où il actionne son dispositif, déplace latéralement la tête de distribution 100 dans sa narine avec le risque que toute une partie du produit soit éjectée de manière non appropriée. Avantageusement, l'élément de l'actionnement 41 comporte une zone de contact arrondie 410 pour coopérer avec cette surface de guidage 50 sur toute sa course d'actionnement. Ceci permet d'éviter au maximum les frottements contre cette surface de guidage afin de limiter au maximum l'impact de la présence de la surface de guidage 50 sur la résistance à l'actionnement du système d'actionnement latéral. Dans le mode de réalisation des figures 1 et 2, la surface de guidage 50 est solidaire de la tête de distribution 100, mais il est entendu que cette surface de guidage 50 pourrait être disposée à un autre endroit plus approprié du dispositif. Comme visible sur les figures, le corps comporte avantageusement un manchon de guidage axial 15 qui reçoit et guide le réservoir 20 lors de son déplacement à l'intérieur du corps pendant l'actionnement du dispositif. Ce manchon de guidage 15 est de préférence disposé en partie basse du corps lorsque le dispositif est en position droite comme représenté sur les figures.

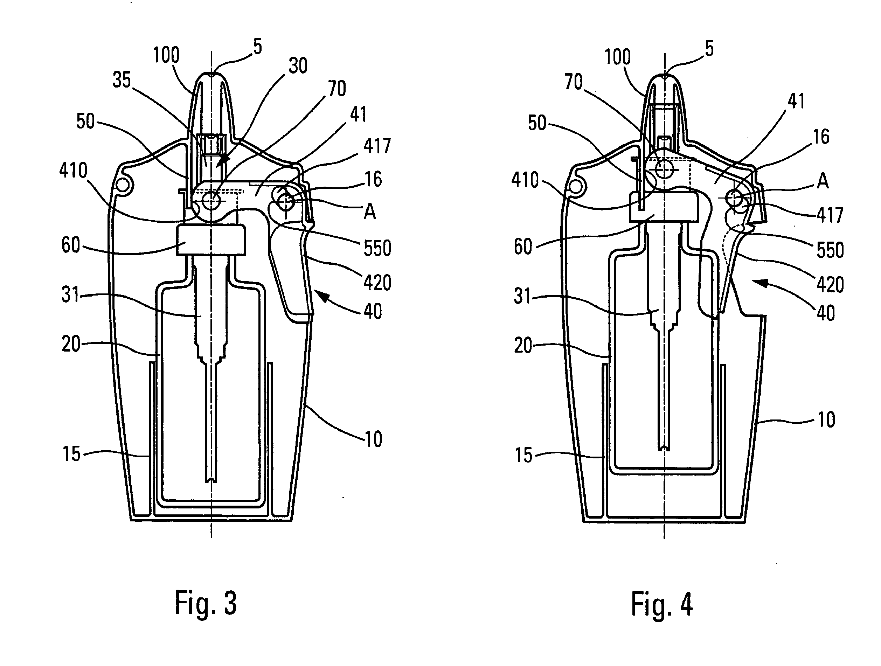

Les figures 3 et 4 représentent un mode de réalisation ne faisant pas partie de l'invention qui diffère par rapport à la réalisation des figures 1 et 2 principalement par l'utilisation d'un système d'actionnement latéral 40 qui ne comporte qu'un seul élément pivotant, à savoir l'élément d'actionnement 41. Dans cette variante, c'est donc l'élément d'actionnement 41 qui comporte la zone d'appui 420 sur laquelle l'utilisateur va appuyer pour actionner le dispositif. La surface de guidage axiale 50 et la coopération entre l'élément d'actionnement 41 et l'unité formée du réservoir et de l'organe de distribution pour réaliser l'actionnement sont similaires à celles décrites par rapport au figure 1 et 2. Par contre, dans ce mode de réalisation, l'élément d'actionnement 41 doit pouvoir se déplacer légèrement par rapport à son axe de pivotement A afin de garantir un contact permanent sur toute sa course d'actionnement avec la surface de guidage axiale 50. Avantageusement ceci peut être réalisé en formant une ouverture oblongue 417 dans l'élément d'actionnement 41, ladite ouverture oblongue 417 venant s'assembler autour d'un plot 16 solidaire du corps 10, ledit plot définissant l'axe de rotation A. Avantageusement, comme visible sur les figures 3 et 4, l'ouverture oblongue est inclinée, vue dans la position droite du dispositif représentée sur les figures, et la largueur de cette ouverture oblongue est environ égale (tout en étant très légèrement supérieure) à celle dudit plot 16, alors que sa longueur est supérieure à celle du plot, ce qui permet donc un coulissement de l'élément d'actionnement 41 le long de cette ouverture oblongue inclinée pendant son pivotement autour de l'axe A. L'intérieur de cette ouverture oblongue 417 forme donc une seconde surface de guidage 550 qui est inclinée par rapport à la surface de guidage 50. La forme de cette seconde surface de guidage 550, ainsi que son inclinaison, dépendent de la forme de la zone de contact arrondie 410 de l'élément d'actionnement 41 qui coopère avec la surface de guidage 50. L'objectif étant d'assurer un appui permanent pour limiter voir supprimer les contraintes radiales exercées sur le corps du dispositif pendant l'actionnement.

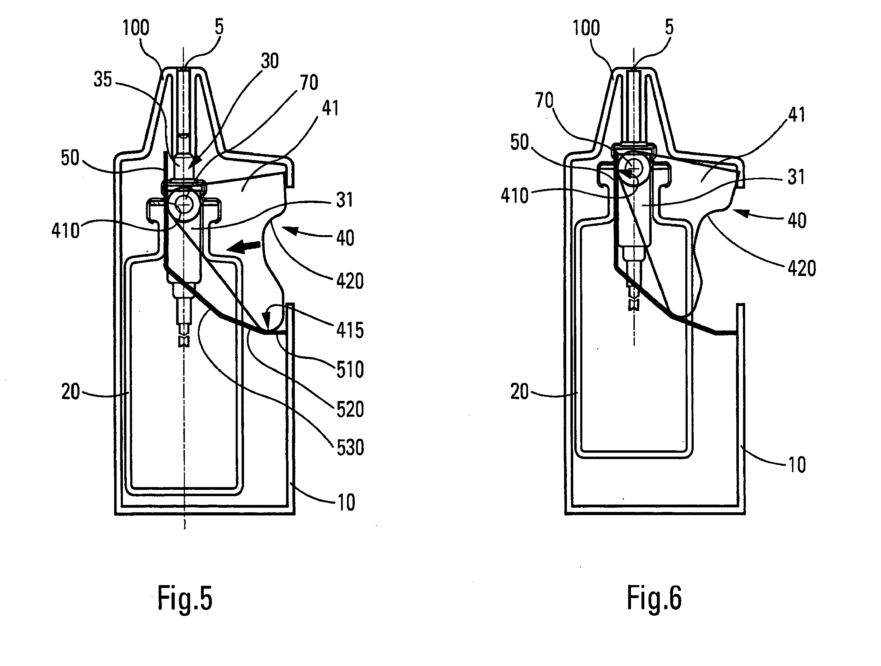

Sur les figures 5 et 6, il est représenté un mode de réalisation qui ne fait pas partie de la présente invention. Dans ce mode de réalisation, le système d'actionnement pivotant latéral 40 comporte un seul élément d'actionnement 41 incorporant la zone d'appui 420 sur laquelle l'utilisateur va appuyer pour réaliser l'actionnement du dispositif, dans le sens de la flèche représentée sur la figure 5. Dans ce mode de réalisation, la surface de guidage 50 n'est pas directement solidaire de la tête de distribution mais est connectée à une autre partie du corps 10 par l'intermédiaire d'une seconde surface de guidage formée dans cet exemple de trois parties 510, 520, 530. Ainsi, dans cette variante, l'axe de rotation de l'élément d'actionnement 41 est formé par le plot 70 solidaire du réservoir 20 et/ou de l'organe de distribution 30 et/ou d'un élément solidaire du réservoir ou de cet organe de distribution. L'élément d'actionnement 41 va donc coopérer d'une part avec la surface de guidage 50 axiale (c'est-à-dire verticale dans la position droite représentée sur les figures) via sa zone de contact arrondie 410, et d'autre part avec la seconde surface de guidage par l'intermédiaire d'une seconde zone de contact 415. De préférence, cette zone de contact est aussi arrondie 415. En se référant plus précisément à l'exemple des figures 5 et 6, la seconde surface de guidage comporte une première partie 510, sensiblement horizontale dans la position droite représentée sur la figure 5, qui coopère avec l'élément d'actionnement 41 lorsque le dispositif est en position de repos. Cette première partie 510 se prolonge par une seconde partie 520 formant un angle par rapport à cette première partie 510, et avec laquelle l'élément d'actionnement 41 va coopérer pendant la course d'actionnement du dispositif. Enfin une troisième partie 530 relie cette seconde partie 520 à la surface de guidage 50 axiale, et l'élément d'actionnement 41 vient coopérer avec cette troisième partie 530 lorsque le dispositif est en position d'actionnement. Bien entendu, cette forme particulière de seconde surface de guidage en trois parties inclinées les unes par rapport aux autres n'est pas obligatoire, et on pourrait envisager une seconde surface de guidage formée d'une seule partie inclinée par rapport à la surface de guidage 50, l'objectif étant ici à nouveau d'assurer une diminution voir une suppression des contraintes radiales exercées par le système d'actionnement latéral lors de l'actionnement.

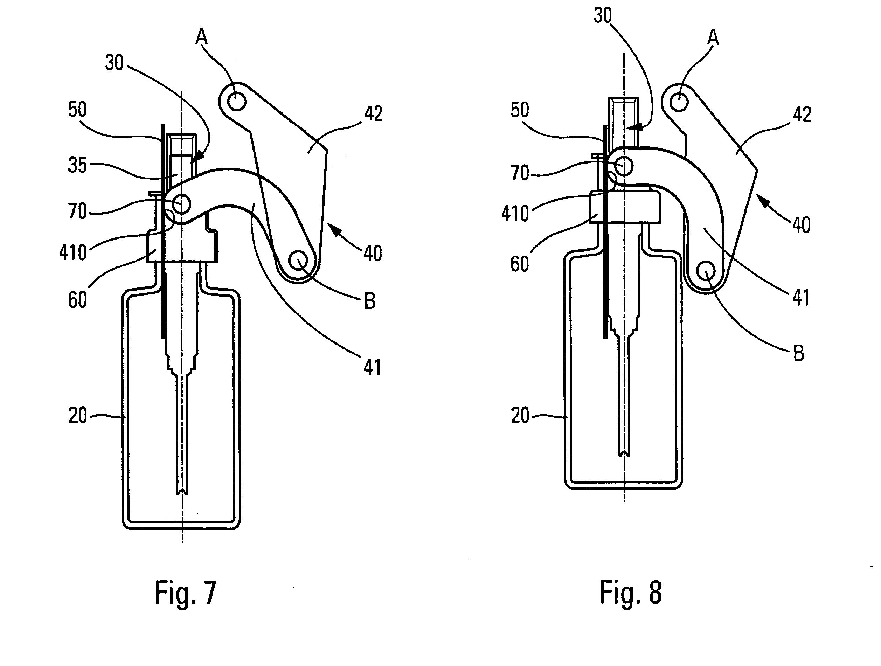

Les figures 7 et 8 représentent un second mode de réalisation de la présente invention. Dans ce mode de réalisation, le système d'actionnement latéral 40 comporte à nouveau deux éléments montés pivotants l'un par rapport à l'autre, de manière similaire au premier mode de réalisation décrit en référence aux figures 1 et 2. Il est à noter que sur ces figures 7 et 8, le dispositif n'est que représenté partiellement. Ainsi, le système d'actionnement latéral 40 comporte un élément d'actionnement 41 coopérant d'un côté avec un élément solidaire du réservoir, tel que la bague de fixation 60, par l'intermédiaire de la projection 70. Cet élément d'actionnement 41 coopère également avec la surface de guidage 50 selon l'invention. Cet élément d'actionnement 41 est ensuite monté pivotant sur un élément intermédiaire d'appui 42 représenté schématiquement sur les figures 7 et 8, lui-même monté pivotant sur le corps autour d'un axe de rotation A. Lorsque l'utilisateur va appuyer sur l'élément d'appui 42, celui-ci va pivoter autour de l'axe A ce qui provoquera un pivotement de l'élément d'actionnement 41 autour de son axe B et un actionnement du dispositif. En principe cet actionnement est similaire à celui des figures 1 et 2, avec toutefois ici l'axe A plus proche de l'orifice de distribution 5 que l'axe B, alors que dans le premier mode de réalisation des figures 1 et 2, l'axe A était plus éloigné de l'orifice de distribution 5 par rapport à l'axe B. Dans le mode de réalisation des figures 7 et 8, l'élément d'actionnement 41 vient donc par en dessous coopérer avec la projection 70 pour actionner le dispositif.

Dans les différents modes de réalisation décrits ci-dessus, l'élément d'actionnement 41 pivote par rapport à la projection 70 avec laquelle il coopère. Dans la mesure où cet élément d'actionnement 41 va coopérer avec une telle projection 70, il comporte de préférence un oeillet monté autour de ladite projection 70 et lui permettant ainsi de pivoter autour de celle-ci. Bien entendu, on peut imaginer en variante que l'élément d'actionnement 41 coopère de manière différente avec une partie d'un élément solidaire du réservoir, par exemple en poussant par en dessous sur un quelconque profil adapté, ou par l'intermédiaire d'une surface de came permettant un déplacement axial de l'organe de distribution 30 lors de l'actionnement latéral du système d'actionnement latéral 40.

D'autres modifications sont envisageables pour l'homme du métier sans sortir du cadre de la présente invention tel que défini par les revendications annexées.

## Revendications

1. Dispositif de distribution de produit fluide, comportant un corps (10), un réservoir (20), une tête de distribution (100) incorporant un orifice de distribution (5), un organe de distribution (30), tel qu'une pompe ou une valve, monté sur ledit réservoir (20), ledit organe de distribution (30) comportant un corps d'organe de distribution (31) et un organe mobile (35) déplacé axialement dans ledit corps d'organe de distribution lors de l'actionnement dudit organe de distribution (30), ledit dispositif comportant en outre un système d'actionnement latéral (40) solidaire dudit corps (10), comportant au moins un élément d'actionnement (41) coopérant avec un élément solidaire dudit réservoir (20), ledit au moins un élément d'actionnement (41) étant mobile entre une position de repos, dans laquelle ledit organe de distribution (30) n'est pas actionné, et une positionnement d'actionnement, dans laquelle ledit organe de distribution (30) est actionné,
ledit système d'actionnement latéral (40) comportant un élément d'appui (42) monté pivotant autour d'un axe (A) sur le corps (10), ledit au moins un élément d'actionnement (41) étant monté pivotant sur ledit élément d'appui (42) autour d'un autre axe (B), ledit corps comportant au moins une surface de guidage (50) dudit au moins un élément d'actionnement (41), ladite surface de guidage (50) étant fixe par rapport à ladite tête de distribution (100) et sensiblement parallèle à l'axe de déplacement de l'organe mobile (35) dans le corps d'organe de distribution (31) lors de l'actionnement dudit organe de distribution (30) **caractérisé en ce que** ledit au moins un élément d'actionnement (41) coopère avec une projection (70) solidaire dudit élément solidaire du réservoir, ladite projection (70) étant solidaire d'une bague (60) assemblée sur ledit réservoir, ladite bague (60) fixant l'organe de distribution (30) sur le réservoir (20).

2. Dispositif selon la revendication 1, dans lequel ledit au moins un élément d'actionnement (41) comporte une zone de contact arrondie (410) coopérant avec ladite surface de guidage (50) sur toute la course d'actionnement.

3. Dispositif selon la revendication 1 ou 2, dans lequel ladite surface de guidage (50) est solidaire de la tête de distribution incorporant l'orifice de distribution (5).

4. Dispositif selon l'une quelconque des revendications précédentes dans lequel ledit au moins un élément d'actionnement (41) comporte un oeillet monté autour de ladite projection (70), ledit élément d'actionnement (41) pivotant autour de ladite projection (70) pendant l'actionnement.

5. Dispositif selon l'une quelconque des revendications précédentes dans lequel ledit élément d'appui (42) comporte une zone d'appui (420) sur laquelle l'utilisateur appuie lors de l'actionnement.

6. Dispositif selon l'une quelconque des revendications précédentes dans lequel ledit axe (A) est plus éloigné de l'orifice de distribution (5) que ledit autre axe (B).

7. Dispositif selon l'une quelconque des revendications 1 à 5 dans lequel ledit axe (A) est plus proche de l'orifice de distribution (5) que ledit autre axe (B).

8. Dispositif selon l'une quelconque des revendications précédentes, dans lequel ledit corps comporte un manchon de guidage axial (15) pour guider le réservoir (20) lors de l'actionnement.

## Patentansprüche

1. Ausgabevorrichtung für ein fluides Produkt, aufweisend einen Körper (10), einen Behälter (20), einen Ausgabekopf (100), der eine Ausgabeöffnung (5) umfasst, eine Ausgabeeinrichtung (30), wie eine Pumpe oder ein Ventil, die auf dem Behälter (20) montiert ist, wobei die Ausgabeeinrichtung (30) einen Körper der Ausgabeeinrichtung (31) sowie eine mobile Einrichtung (35) aufweist, die bei Betätigung der Ausgabeeinrichtung (30) axial in dem Körper der Ausgabeeinrichtung verschiebbar ist, wobei die Vorrichtung des Weiteren ein seitliches Betätigungssystem (40) aufweist, das mit dem Körper (10) in einem Stück gebildet ist und mindestens ein Betätigungselement (41) aufweist, das mit einem mit dem Behälter (20) in einem Stück gebildeten Element zusammenwirkt, wobei das mindestens eine Betätigungselement (41) zwischen einer Ruheposition, in der die Ausgabeeinrichtung (30) nicht betätigt wird, und einer Betätigungsposition, in der die Ausgabeeinrichtung (30) betätigt wird, beweglich ist,
wobei das seitliche Betätigungssystem (40) ein Drückelement (42) aufweist, welches schwenkbar um eine Achse (A) auf dem Körper (10) montiert ist, wobei das mindestens eine Betätigungselement (41) schwenkbar um eine andere Achse (B) auf dem Drückelement (42) montiert ist, wobei der Körper mindestens eine Führungsfläche (50) des mindestens einen Betätigungselements (41) aufweist, wobei die Führungsfläche (50) bei Betätigung der Ausgabeeinrichtung (30) in Bezug auf den Ausgabekopf (100) fixiert und im Wesentlichen parallel zur Verschiebungsachse der mobilen Einrichtung (35) in dem Körper der Ausgabeeinrichtung (31) ist, **dadurch gekennzeichnet, dass** das mindestens eine Betätigungselement (41) mit einem Vorsprung (70) zusammenwirkt, der mit dem einstückig mit dem Behälter gebildeten Element einstückig ist, wobei der Vorsprung (70) mit einem Ring (60) einstückig gebildet ist, der auf dem Behälter angebracht ist, wobei der Ring (60) die Ausgabeeinrichtung (30) auf dem Behälter (20) fixiert.

2. Vorrichtung nach Anspruch 1, wobei das mindestens eine Betätigungselement (41) einen abgerundeten Kontaktbereich (410) umfasst, der über den gesamten Betätigungsverlauf mit der Führungsfläche (50) zusammenwirkt.

3. Vorrichtung nach Anspruch 1 oder 2, wobei die Führungsfläche (50) mit dem Ausgabekopf, der die Ausgabeöffnung (5) umfasst, einstückig gebildet ist.

4. Vorrichtung nach einem der vorhergehenden Ansprüche, wobei das mindestens eine Betätigungselement (41) eine Öse aufweist, die um den Vorsprung (70) montiert ist, wobei das Betätigungselement (41) bei Betätigung um den Vorsprung (70) verschwenkt wird.

5. Vorrichtung nach einem der vorhergehenden Ansprüche, wobei das Drückelement (42) einen Drückbereich (420) aufweist, auf den der Benutzer bei Betätigung drückt.

6. Vorrichtung nach einem der vorhergehenden Ansprüche, wobei die Achse (A) von der Ausgabeöffnung (5) weiter entfernt ist als die andere Achse (B).

7. Vorrichtung nach einem der Ansprüche 1 bis 5, wobei die Achse (A) näher an der Ausgabeöffnung (5) ist als die andere Achse (B).

8. Vorrichtung nach einem der vorhergehenden Ansprüche, wobei der Körper eine axiale Führungshülse (15) aufweist, um den Behälter (20) bei der Betätigung zu führen.

## Claims

1. A fluid dispenser device comprising: a body (10); a reservoir (20); a dispenser head (100) incorporating a dispenser orifice (5); a dispenser member (30), such as a pump or a valve, that is mounted on said reservoir (20), said dispenser member (30) comprising a dispenser-member body (31) and a movable member (35) that moves axially in said dispenser-member body while said dispenser member (30) is being actuated; said device further comprises a lateral actuator system (40) that is secured to said body (10), said lateral actuator system comprising at least one actuator element (41) that co-operates with an element that is secured to said reservoir (20), said at least one actuator element (41) being movable between a rest position in which said dispenser member (30) is not actuated, and an actuated position in which said dispenser member (30) is actuated, said lateral actuator system (40) including a presser element (42) that is mounted on the body (10) to pivot about an axis (A), said at least one actuator element (41) being mounted on said presser element (42) to pivot about an axis (B), said body (10) including at least one guide surface (50) for guiding said at least one actuator element (41), said guide surface (50) being stationary relative to said dispenser head (100), and substantially parallel to the movement axis of the movable member (35) in the dispenser-member body (31) while said dispenser member (30) is being actuated,
the device being **characterized in that** said at least one actuator element (41) co-operates with a projection (70) that is secured to said element that is secured to the reservoir, said projection (70) being secured to a ring (60) that is assembled on said reservoir (20), said ring (60) fastening the dispenser member (30) on the reservoir (20).

2. A device according to claim 1, wherein said at least one actuator element (41) includes a rounded contact zone (410) that co-operates with said guide surface (50) over the entire actuation stroke.

3. A device according to claim 1 or claim 2, wherein said guide surface (50) is secured to the dispenser head incorporating the dispenser orifice (5).

4. A device according to any preceding claim, wherein said at least one actuator element (41) includes an eyelet that is mounted around said projection (70), said actuator element (41) pivoting about said projection (70) during actuation.

5. A device according to any preceding claim, wherein said presser element (42) includes a presser zone (420) against which the user presses during actuation.

6. A device according to any preceding claim, wherein said axis (A) is further from the dispenser orifice (5) than said axis (B).

7. A device according to any one of claims 1 to 5, wherein said axis (A) is closer to the dispenser orifice (5) than said axis (B).

8. A device according to any preceding claim, wherein said body includes an axial guide sleeve (15) for guiding the reservoir (20) during actuation.
